(19) Europäisches Patentamt
European Patent Office
Office européen des brevets

(11) EP 0 421 230 B1

(12) EUROPÄISCHE PATENTSCHRIFT

(45) Veröffentlichungstag und Bekanntmachung des Hinweises auf die Patenterteilung:
**27.03.1996 Patentblatt 1996/13**

(51) Int. Cl.[6]: **C08G 65/26**, C08G 65/32, C08G 65/14, C09D 171/00, C08L 63/00

(21) Anmeldenummer: **90118300.4**

(22) Anmeldetag: **24.09.1990**

(54) **Kationisch härtbare Oxyalkylenether, Verfahren zu ihrer Herstellung und ihre Verwendung als Vergussmassen, Beschichtungsmittel oder als reaktive Verdünnungsmittel für Epoxidharze**

Cationically curable oxyalkylene ethers, process for their preparation and their use as casting mass, coatings or as reactive diluents for epoxy resins

Ethers d'oxyalkylène durcissables cationiquement, leur procédé de préparation et leur utilisation en tant que masses à mouler, matières de revêtements ou diluants réactifs pour des résines époxydes

(84) Benannte Vertragsstaaten:
**BE DE FR GB IT NL**

(30) Priorität: **06.10.1989 DE 3933420**

(43) Veröffentlichungstag der Anmeldung:
**10.04.1991 Patentblatt 1991/15**

(73) Patentinhaber: **Th. Goldschmidt AG**
**D-45127 Essen (DE)**

(72) Erfinder:
- **Weitemeyer, Christian, Dr.**
  **D-4300 Essen 1 (DE)**
- **Döhler, Hardi**
  **D-4300 Essen 14 (DE)**

(56) Entgegenhaltungen:
**EP-A- 0 244 841** **US-E- 31 469**

- **JOURNAL OF THE CHEMICAL SOCIETY, PERKIN TRANSACTIONS 1., Nr. 1, 1979, Letchworth (GB); Seiten 712-718; R. GIGG; 'The Allyl Ether as a Protecting Group in Carbohydrate Chemistry'**
- **FARBE + LACK, 1987, Hannover (DE); Seiten 803-807; J.V. CRIVELLO: 'Geschwindigkeitsbetimmende Faktoren bei der kationischen UV-Härtung'**

Bemerkungen:
Die Akte enthält technische Angaben, die nach dem Eingang der Anmeldung eingereicht wurden und die nicht in dieser Patentschrift enthalten sind.

EP 0 421 230 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft kationisch härtbare, gegebenenfalls substituierte, Vinylgruppen enthaltende Oxyalkylenether. Die Erfindung betrifft ferner Verfahren zur Herstellung dieser Oxyalkylenether und die Verwendung dieser Verbindungen als photochemisch härtbare Oligomere in Vergußmassen oder Beschichtungsmitteln oder als reaktive Verdünnungsmittel für Epoxidharze.

Neben den bei UV-Einwirkung härtenden radikalisch polymerisierenden Systemen sind in den letzten Jahren kationisch härtbare, Epoxy- oder Vinylverbindungen enthaltende Systeme entwickelt worden, bei denen die Härtung insbesondere durch Diaryliodonium- und Triarylsulfoniumsalze initiiert wird. Der Vorteil der kationisch härtenden Systeme liegt in der Unempfindlichkeit der Härtungsreaktion gegen Luftsauerstoff, die rasche Filmhärtung und die Umweltfreundlichkeit dieser Systeme.

Es gibt hierzu eine umfangreiche Patentliteratur. Die kationische Härtung von Epoxyverbindungen mit Oniumsalzen der V., VI. und VII. Hauptgruppe ist u. a. in den DE-Patentschriften 25 18 656, 25 18 652 und 25 18 639 beschrieben. Die kationische Härtung von Vinylmonomeren ist Gegenstand der US-Patentschriften 4 617 238, 4 518 788 und 4 705 887.

Dabei haben die UV-härtbaren Vinyletherverbindungen aufgrund ihrer schnellen Härtbarkeit, der wirtschaftlichen Verarbeitbarkeit und ihrer Umweltfreundlichkeit besondere Beachtung gefunden. Derartige Vinyletherverbindungen lassen sich auf verschiedene Weise herstellen. In einem übersichtsreferat über "Geschwindigkeitsbestimmende Faktoren bei der kationischen UV-Härtung" in der Zeitschrift Farbe und Lack, 1987, S. 803-807, werden folgende Reaktionsmöglichkeiten angegeben:

1. Basenkatalysierte Acetylenaddition an Diole nach Reppe

$$HO\text{-}R\text{-}OH + 2\ HC{\equiv}CH \xrightarrow{NaOH} R\text{-}(O\text{-}CH{=}CH_2)_2$$

2. Phasentransferkatalysierte Kondensation von 2-Chlorethylvinylether mit Diolen

$$HO\text{-}R\text{-}OH + 2\ Cl\text{-}CH_2\text{-}CH_2O\text{-}CH{=}CH_2 \xrightarrow[(C_4H_9)_4N^+Br^-]{NaOH} R\text{-}(O\text{-}CH_2\text{-}CH_2\text{-}O\text{-}CH{=}CH_2)_2$$

3. Katalysierte Umlagerung der Bis-allylether in die entsprechenden Bis-propenylether

$$R\text{-}(O\text{-}CH_2\text{-}CH{=}CH_2)_2 \xrightarrow{[(C_6H_5)_3P]_3RuCl_2} R\text{-}(O\text{-}CH{=}CH\text{-}CH_3)_2$$

Die Addition nach Reppe ist unter wirtschaftlichen Gesichtspunkten nicht realisierbar. Bei der zweiten Reaktion benötigt man 2-Chlorethylether, dessen Einsatz aus physiologischen Gründen unerwünscht ist. Allen drei Reaktionsweisen ist gemeinsam, daß je OH-Gruppe des Alkohols immer nur eine Vinylethergruppe in das Molekül eingeführt wird.

Der vorliegenden Erfindung liegt die Aufgabe zugrunde, Verbindungen zu finden, welche eine nahezu beliebig große Anzahl von, gegebenenfalls substituierten, Vinylethergruppen aufweisen, um die Härtungs- und Vernetzungseigenschaften beeinflussen zu können.

Die Verbindungen sollen außerdem einfach und aus physiologisch möglichst unbedenklichen Ausgangsverbindungen herstellbar sein.

Gegenstand der Erfindung sind neue kationisch härtbare Oxyalkylenether der allgemeinen durchschnittlichen Formel

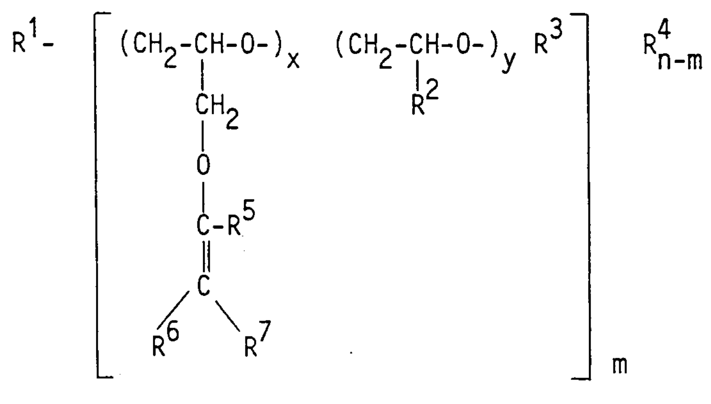

I

| | |
|---|---|
| $R^1$ | = n-wertiger Rest einer zur Anlagerung von Oxiranen befähigten Verbindung $(R^1)H_n$ mit n aktiven Wasserstoffatomen oder einwertiger Alkyl- oder Arylrest, $n \geqq 1$, |
| $R^2$ | = Wasserstoff-, Kohlenwasserstoff- oder $R^3OCH_2$-Rest, |
| $R^3$, $R^4$ | = Wasserstoff-, Kohlenwasserstoff- oder Acylreste, wobei die Reste $R^1$, $R^2$, $R^3$ und $R^4$ im Molekül unterschiedliche Bedeutung haben können, |
| $R^5$, $R^6$, $R^7$ | = Wasserstoff- oder Alkylreste mit 1 bis 8 Kohlenstoffatomen oder $R^5$ und $R^7$ oder $R^6$ und $R^7$ gemeinsam Bestandteil eines cyclischen, nichtaromatischen Kohlenwasserstoffrestes mit 5 oder 6 Kohlenstoffatomen, |
| m | mindestens =1 und höchstens = n |
| x | im durchschnittlichen Molekül = 1 bis 100, |
| y | im durchschnittlichen Molekül = 0 bis 100, wobei $1 \leqq x + y < 150$ ist, und |

wobei Oxyalkylenether ausgenommen sind, bei denen $R^1$ = $-O-n-C_{16}H_{33}$, $R^3$ = H oder $-CH_2-CH=CH-CH_3$, $R^5 = R^6$ = H, $R^7 = CH_3$ und x = 1, y = 0; m = 1 ist.

$R^1$ ist ein n-wertiger Rest, der sich von einer zur Anlagerung befähigten Verbindung $(R^1)H_n$ mit n aktiven Wasserstoffatomen herleitet oder ist ein einwertiger Alkyl- oder Arylrest.

Als zur Anlagerung von Oxiranen befähigte Verbindung $(R^1)H_n$ sind Wasser, Ammoniak, ein ein- oder mehrwertiger Alkohol, ein ein- oder mehrwertiges Phenol, eine ein- oder mehrwertige Carbonsäure oder ein ein- oder mehrwertiges Amin bevorzugt.

Beispiele solcher Verbindungen sind:

| Verbindung $(R^1)H_n$ | Rest $R^1$ | n |
|---|---|---|
| $CH_3OH$ | $CH_3O-$ | 1 |
| $H_2O$ | $-O-$ | 2 |
| $HOCH_2CH_2OH$ | $-OCH_2CH_2O-$ | 2 |
| $CH_2OH$–$CHOH$–$CH_2OH$ | $CH_2O-$–$CHO-$–$CH_2O-$ | 3 |
| $HO-C_6H_4-OH$ | $-O-C_6H_4-O-$ | 2 |
| $HOOC-CH_2CH_2CH_2CH_2-COOH$ | $-OOC-CH_2-CH_2-CH_2-CH_2-COO-$ | 2 |
| $C_2H_5NH_2$ | $C_2H_5N<$ | 2 |

Der Rest $R^1$ ist somit formal der Rest, welcher entsteht, wenn man von der Verbindung $(R^1)H_n$ n Wasserstoffreste abspaltet.

Weitere Beispiele von Verbindungen mit einem oder mehreren aktiven Wasserstoffatomen können der Literatur, zum Bespiel der EP-OS 0 061 822 entnommen werden.

Besonders bevorzugte Verbindungen $(R^1)H_n$ sind einwertige gesättigte aliphatische Alkohole mit 1 bis 13 Kohlenstoffatomen, wie Methanol, Ethanol, Propanol, Butanol, i-Butanol, Hexanol, Decanol, Tridecanol.

Ferner sind Verbindungen $(R^1)H_n$ einwertige ungesättigte Alkohole mit 3 bis 13 Kohlenstoffatomen bevorzugt, wie der Allylalkohol, Oleylalkohol, 5-Hexen-1-ol, 3-Methyl-3-buten-1-ol, 2-Hydroxymethyl-5-norbornen.

Außerdem sind zwei- bis sechswertige aliphatische Alkohole mit 2 bis 6 Kohlenstoffatomen bevorzugt. Hierzu gehören Ethylenglykol, Glycerin, Sorbit, Trimethylolpropan, Pentaerythrit, Dipentaerythrit.

Ferner sind bevorzugte Verbindungen der Formel $(R^1)H_n$ aliphatische gesättigte oder ungesättigte ein- oder mehrwertige Carbonsäuren, wie Essigsäure, Laurinsäure, Ölsäure, Fumarsäure, Maleinsäure, Bernsteinsäure, Adipinsäure, Phthalsäure, Terephthalsäure.

Schließlich sind als Verbindungen $(R^1)H_n$ noch die aliphatischen Mono- und Diamine mit 1 bis 7 Kohlenstoffatomen zu nennen, wie mono-oder dialkylsubstituiertes Ethylendiamin, 1,3-Propylendiamin, 1,3-bzw. 1,4-Butylendiamin, Allylamin, 2-Methylallylamin, Phenylendiamine, Hexamethylendiamine.

Der Rest $R^1$ kann auch ein einwertiger Alkyl- oder Arylrest sein. Bevorzugt ist $R^1$ dann ein niedriger Alkylrest, insbesondere der Methylrest oder der Phenylrest.

$R^2$ ist ein Wasserstoff-, Kohlenwasserstoff- oder $R^3OCH_2$-Rest. Vorzugsweise ist $R^2$ ein Wasserstoff- oder niedriger Alkylrest mit 1 bis 4 Kohlenstoffatomen, besonders bevorzugt ein Methyl- oder Ethylrest.

$R^3$ ist ein Wasserstoff-, Kohlenwasserstoff- oder Acylrest, vorzugsweise ein Wasserstoff- oder niedriger Alkylrest mit 1 bis 4 Kohlenstoffatomen. Als Acylrest ist der Acetylrest bevorzugt.

$R^4$ ist ein Wasserstoff-, Kohlenwasserstoff- oder Acylrest. Bevorzugt ist $R^4$ ein Wasserstoffrest oder ein Alkylrest mit 1 bis 4 Kohlenstoffatomen. Als Acylrest ist wiederum der Acetylrest bevorzugt.

Im polymeren Molekül können die einzelnen Reste $R^1$, $R^2$, $R^3$ und $R^4$ jeweils unterschiedliche Bedeutung haben.

$R^5$, $R^6$ und $R^7$ sind Wasserstoff- oder Alkylreste mit 1 bis 8 Kohlenstoffatomen. Vorzugsweise sind die Reste Wasserstoffreste oder niedere Alkylreste mit insbesondere 1 bis 4 Kohlenstoffatomen. Es können jedoch auch $R^5$ und $R^7$ bzw. $R^6$ und $R^7$ gemeinsam Bestandteil eines cyclischen, nichtaromatischen Kohlenwasserstoffrestes mit 5 oder 6 Kohlenstoffatomen sein. Vorzugsweise sind in diesem Fall die Reste $R^5$ und $R^7$ Bestandteil eines solchen cyclischen Restes, wobei $R^6$ ein Wasserstoffrest ist.

Sind die Reste $R^6$ und $R^7$ verschieden, besteht die Möglichkeit der Ausbildung cis-, trans-isomerer Verbindungen. Beide Formen sind jedoch im Sinne der Erfindung brauchbar.

m hat einen Zahlenwert von mindestens 1 und ist höchstens gleich n. Ist n gleich 1, entfällt der Rest $R^4$.

x gibt die Anzahl der Oxyalkyleneinheiten mit seitenständig gebundenen, gegebenenfalls substituierten, Vinylresten an. x hat dabei im durchschnittlichen Molekül einen Wert von 1 bis 100. Vorzugsweise soll das Produkt m . x größer als 1 sein.

y gibt die Anzahl der Oxyalkyleneinheiten, welche frei von Vinylresten sind, an. y hat dabei im durchschnittlichen Molekül einen Wert von 0 bis 100, wobei die Beziehung $1 \leqq x + y < 150$ erfüllt sein soll. Das Produkt aus m . y beträgt vorzugsweise 0 bis 25.

Beispiele erfindungsgemäßer kationisch härtbarer Oxyalkylenether sind durch die folgenden Formeln wiedergegeben:

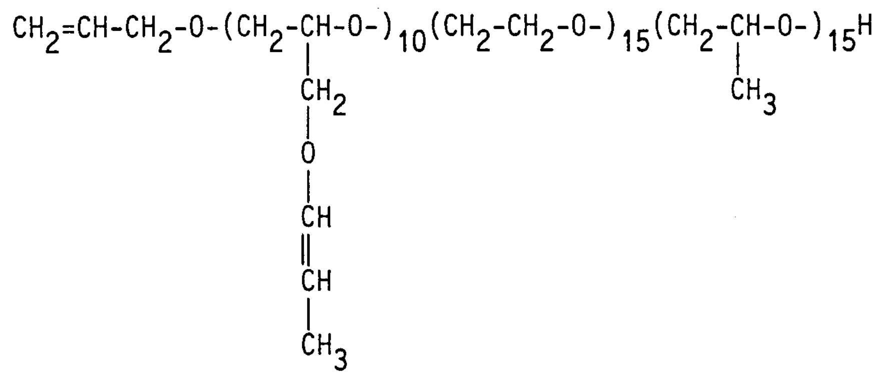

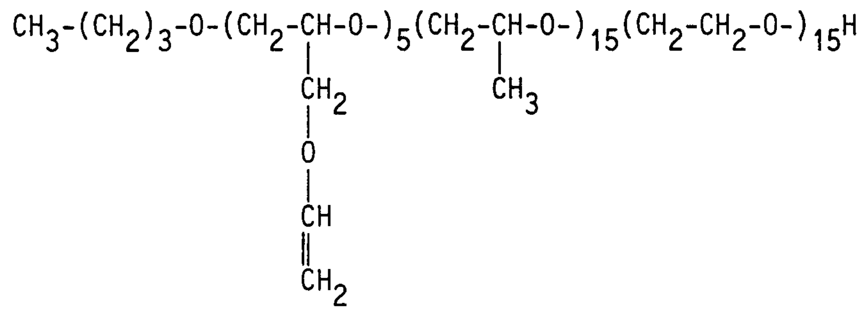

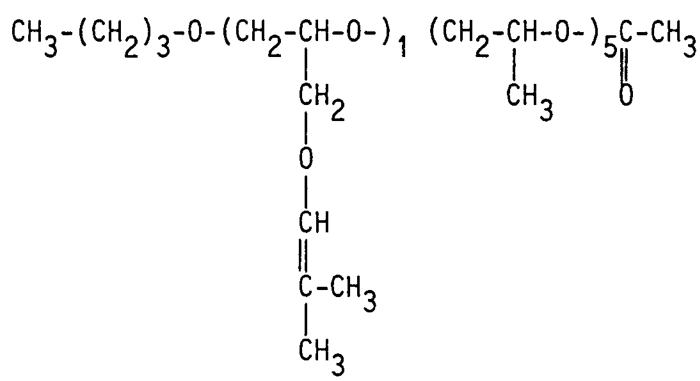

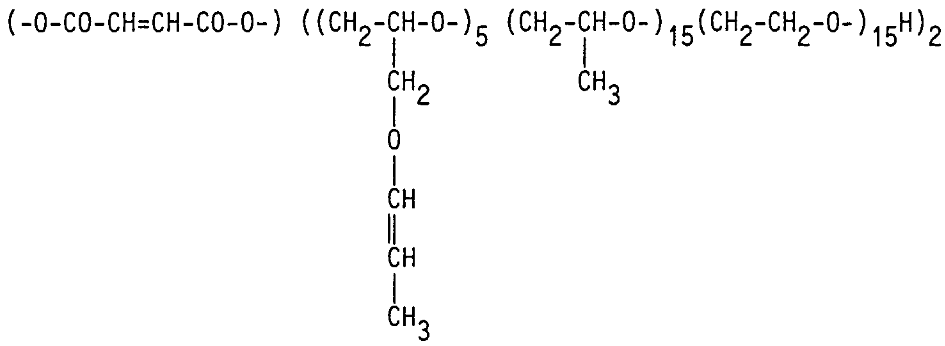

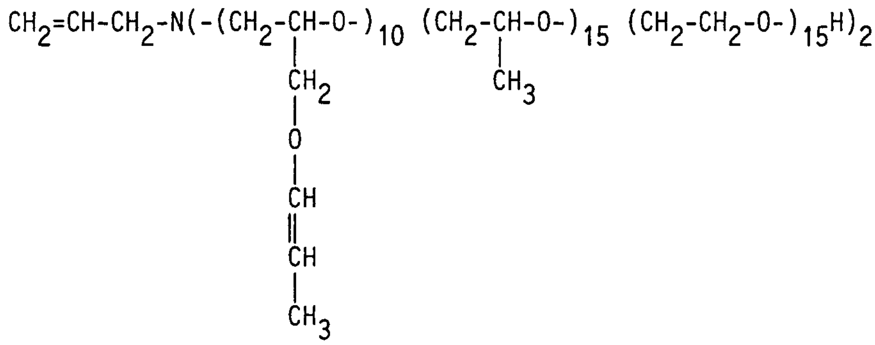

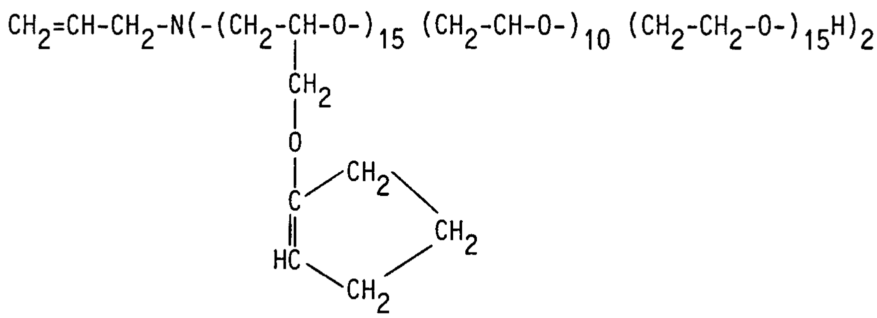

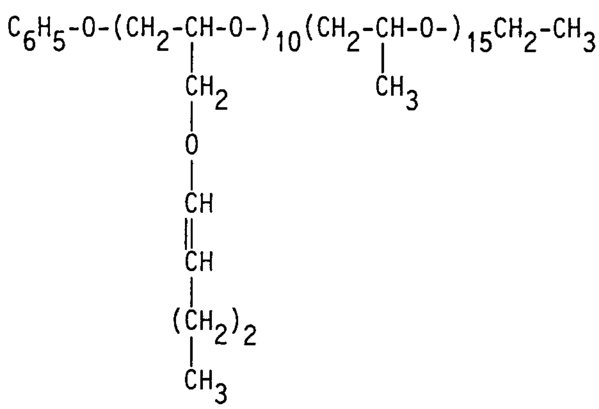

Ein weiterer Gegenstand der vorliegenden Erfindung besteht in der Herstellung der neuen härtbaren Polyalkylenether.

Ein besonders bevorzugtes Verfahren ist dadurch gekennzeichnet, daß man
x . m Mol Monomere der allgemeinen Formel

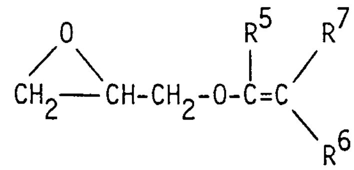

und gegebenenfalls y . m Mol Monomere der allgemeinen Formel

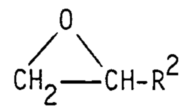

an 1 Mol einer Verbindung der allgemeinen Formel $(R^1)H_n$ statistisch oder blockweise unter alkalischen oder im wesentlichen neutralen Bedingungen anlagert und gegebenenfalls die erhaltenen Verbindungen ganz oder teilweise mit Alkylhalogeniden der Formel $R^3X$ verethert oder mit Carbonsäureanhydriden der Formel $(R^3CO)_2O$ oder mit Acylhalogeniden der Formel $R^3COX$ verestert, wobei X ein Halogenatom ist.

Die Addition der Alkylenoxide an die Verbindungen mit aktivem Wasserstoffatom erfolgt in an sich bekannter Weise, vorzugsweise unter einem Druck von 0,1 bis 10 bar, gegebenenfalls unter Schutzgas, in einem geschlossenen System. Als Katalysatoren verwendet man bevorzugt Alkali-oder Erdalkalialkoholate, insbesondere die entsprechenden Salze des Methanols oder Ethanols sowie Alkalihydroxide, wie KOH oder NaOH.

Die Anlagerungsreaktion verläuft in der Regel bei Temperaturen von 50 bis 200 °C, insbesondere 80 bis 150 °C.

Man kann die Addition auch unter Verwendung von Alkyllithiumverbindungen durchführen. Die Reaktion verläuft nach dem Schema:

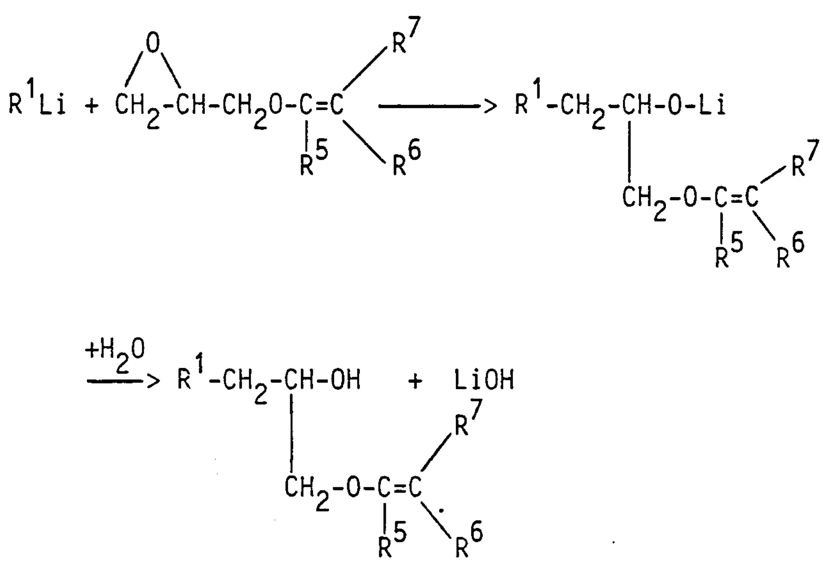


Bei dieser Reaktion entstehen immer Verbindungen, bei denen n einen Wert von 1 hat und somit der Rest $R^4$ entfällt.

Beispiele geeigneter Alkylenoxide mit und ohne Vinylreste sind:

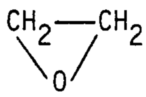; 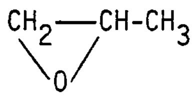; 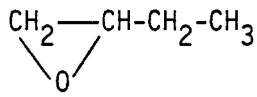; 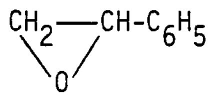;

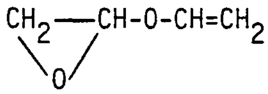; 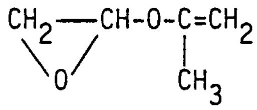; 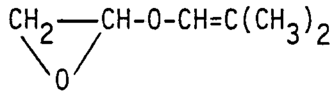;

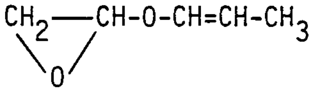; 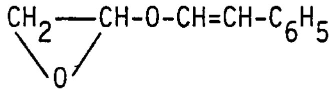; 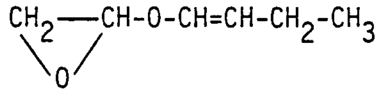

Ein weiteres Herstellungsverfahren ist dadurch gekennzeichnet, daß man Verbindungen der allgemeinen durchschnittlichen Formel

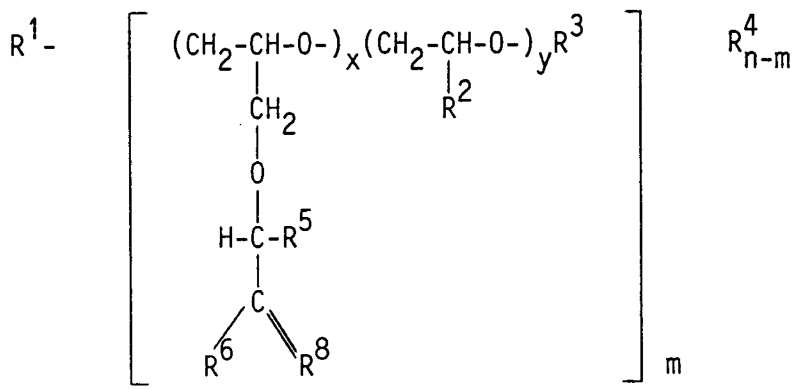

II

wobei $R^1$, $R^2$, $R^3$, $R^4$, $R^5$ und $R^6$ die bereits angegebenen Bedeutungen und x, y, n und m die bereits angegebenen Werte haben und der Rest $R^8$ eine Alkylengruppe darstellt, in an sich bekannter Weise umlagert.

Die Reste und Indices haben die bereits angegebene Bedeutung. $R^8$ ist ein Kohlenwasserstoffrest mit 1 bis 8 Kohlenstoffatomen und zwei freien Bindungen an einem Kohlenstoffatom, wie der Rest $=CH_2$, $=CH-CH_3$, $=CH-CH_2-CH_3$.

Die Umlagerung der Doppelbindung in $\alpha$-Stellung zum an den Ethersauerstoff gebundenen Kohlenstoffatom (Umlagerung zur gegebenenfalls substituierten Propenylverbindung) erfolgt in an sich bekannter Weise. Vorzugsweise nimmt man die Umlagerung durch Einwirkung von Alkalialkoholaten oder Metallkomplexen oder Übergangsmetalle enthaltenen Katalysatoren bei Temperaturen von etwa 25 bis 170 °C, gegebenenfalls in polaren Lösungsmitteln, vor.

Die erfindungsgemäßen Verfahren haben den Vorteil, daß sie in einfacher Weise unter Verwendung gut zugänglicher Ausgangsverbindungen ablaufen, wobei eine Verwendung der toxischen Chlorethylverbindungen vermieden wird.

Ein weiterer Gegenstand der vorliegenden Erfindung besteht in der Verwendung der erfindungsgemäßen Verbindungen. Die erfindungsgemäßen Verbindungen härten nach Zusatz von an sich bekannten Photoinitiatoren bei Einwirkung von UV-Strahlung aus. Beispiele geeigneter Initiatoren sind die Oniumsalze von insbesondere Verbindungen der V. bis VII. Hauptgruppe, wie die arylsubstituierten Phosphonium-, Sulfonium-und Iodoniumsalze starker Säuren oder nicht-salzartige Verbindungen, wie die Ketosulfone.

Beispiele für Photoinitiatoren vom Typ der Oniumsalze sind:

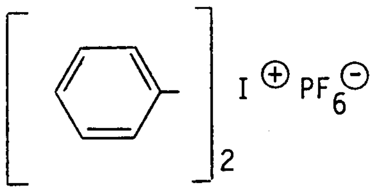
;
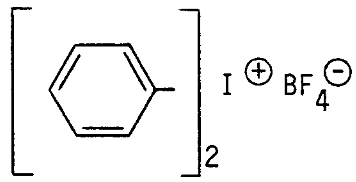

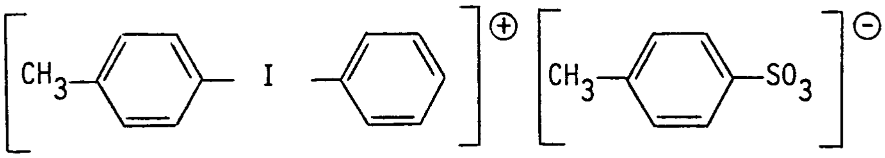

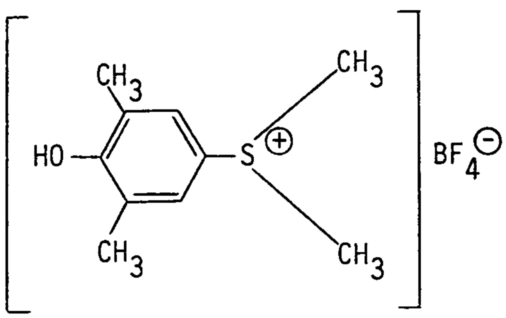
;
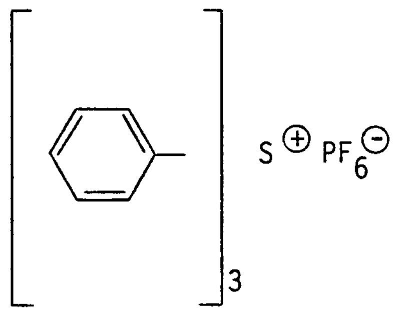

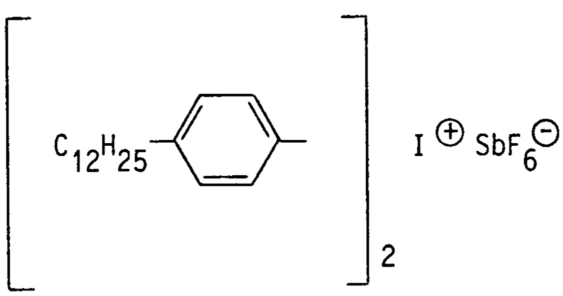
;
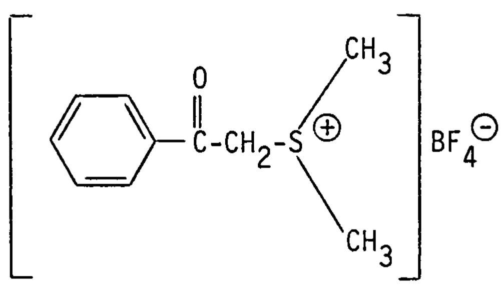

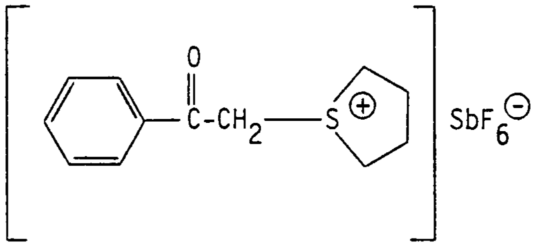 ; 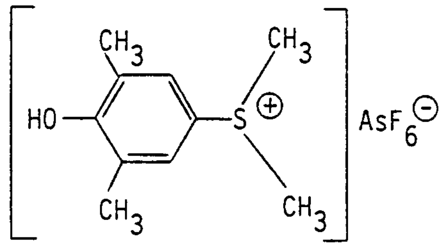

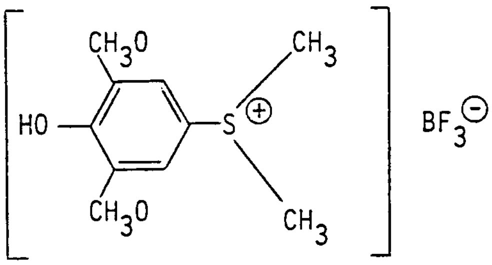

Besonders bevorzugte Initiatoren sind:

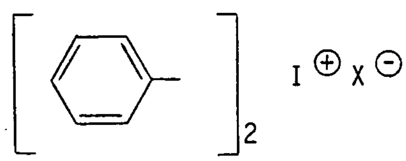 ; 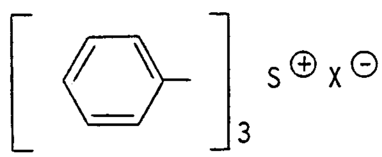

$X^{\ominus} = PF_6^{\ominus}$ ; $BF_4^{\ominus}$ ; $SbF_6^{\ominus}$

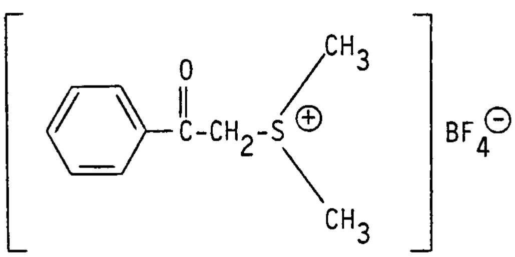 ; 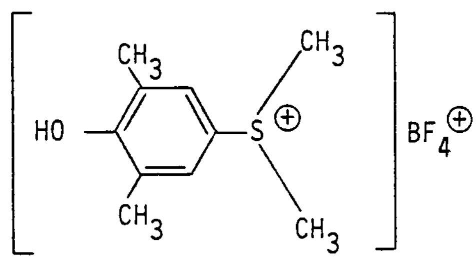

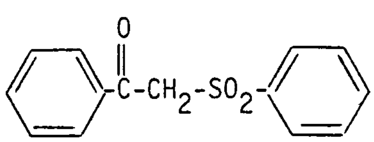 ; 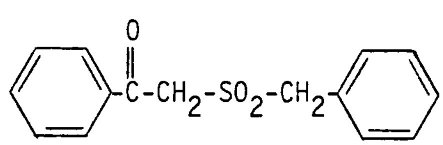

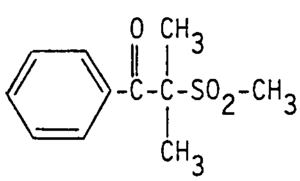 ; 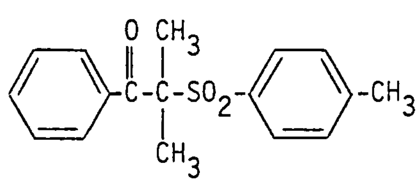

Des weiteren ist eine Hitzehärtung unter Verwendung von Oniumsalzen mit organischen Oxidationsmitteln oder löslichen Kupfersalzen oder Chelaten, wie in US-PS 4 173 511 beschrieben, möglich. Eine andere Möglichkeit die Härtung herbeizuführen, besteht in der Verwendung von Verbindungen, die bei Temperaturerhöhung Säuren oder Lewissäuren freisetzen, wie Sulfonsäuresalze, besonders Aminsalze, Sulfonsäureester und Aminkomplexe von Lewissäuren, wie z. B. Bortrifluoridtriethylaminkomplex.

Die Initiatoren werden den erfindungsgemäßen Verbindungen in Mengen von etwa 0,5 bis 10 Gew.-% zugesetzt.

Die erfindungsgemäßen Verbindungen härten in kürzester Zeit (Bruchteile von Sekunden bis zu einigen Sekunden) zu klebfreien, flexiblen oder harten Produkten aus. Sie können deshalb in vorteilhafter Weise als Vergußmassen, z. B. für elektronische Bauteile, als Beschichtungsmaterial für flächige Träger und als reaktive Verdünnungsmittel für Epoxyharze verwendet werden.

Gegenüber den bekannten Vinylverbindungen des Standes der Technik haben sie den Vorteil der Anordnung einer praktischen beliebigen Anzahl von, gegebenenfalls substituierten, Vinylresten längs der Kette des polymeren Moleküls. Hierdurch kann die Aushärtungsgeschwindigkeit und die Vernetzungsdichte den sich aus der jeweiligen Verwendung ergebenden Anforderungen angepaßt werden. Ein weiterer Vorteil besteht darin, daß die Verbindungen, wenn $R^3$ und/oder $R^4$ ein Wasserstoffatom bedeuten, weiteren Umsetzungen, z. B. mit Isocyanaten, zugänglich sind.

Bei der Verwendung können die Verbindungen mit üblichen Zusatzstoffen, wie Modifizierungsmitteln, Pigmenten, Füllstoffen, Flammschutzmitteln und dergleichen vermischt werden.

In den folgenden Beispielen werden die bevorzugten Herstellungsverfahren noch weiter erläutert und die Eigenschaften der erfindungsgemäßen Verbindungen durch anwendungstechnische Versuche gezeigt:

Beispiel 1

In einem Rührbehälter werden 66,6 g (0,9 Mol) Butanol mit 7 g (0,1 Mol) Kaliummethylat vorgelegt und mit einer Stickstoffatmosphäre überlagert. Bei 100 °C werden 114 g (1 Mol) Propenylglycidether so zugetropft, daß die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 175 g (95 % der Theorie) eines leicht gelblichen niedrigviskosen Produktes erhalten, das nach dem $^1$H-NMR die durchschnittliche Formel

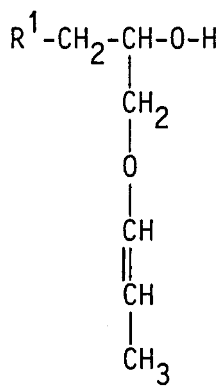

mit $R^1$ = zu 90 % Butanolrest und
zu 10 % Methanolrest

besitzt.

Beispiel 2

In einem Rührbehälter werden 66,6 g (0,9 Mol) Butanol mit 7 g (0,1 Mol) Kaliummethylat vorgelegt und mit einer Stickstoffatmosphäre überlagert. Bei 100 °C werden 342 g (3 Mol) Propenylglycidether so zugetropft, daß die Temperatur 120 °C nicht überschreitet. Das Reaktionsgemisch wird noch 3 Stunden bei 100 °C gerührt, mit 50 g feingepulverter KOH versetzt und solange Methylchlorid eingeleitet, bis kein Methylchlorid mehr vom Reaktionsgemisch aufgenommen wird. Anschließend wird mit 50 %iger Phosphorsäure neutralisiert. Nach Filtration wird das Produkt unter vermindertem Druck bis 100 °C destilliert. Es werden 408 g (96 % der Theorie) eines leicht gelblichen niedrigviskosen Produktes erhalten, das nach dem $^1$H-NMR die durchschnittliche Formel

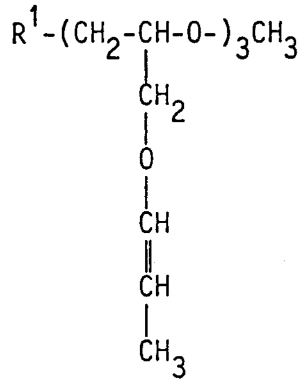

mit

$R^1$ = zu 90 % Butanolrest und
zu 10 % Methanolrest

besitzt.

Beispiel 3

In einem Druckbehälter werden 66,6 g (0,9 Mol) Butanol mit 7 g (0,1 Mol) Kaliummethylat vorgelegt und mit einer Stickstoffatmosphäre von 2 bar belegt. Bei 100 °C wird gleichzeitig 580 g (10 Mol) Propylenoxid und 342 g (3 Mol) Propenylglycidether so eingeleitet bzw. zugetropft, daß der Druck 5 bar und die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 932 g (94 % der Theorie) eines leicht gelblichen niedrigviskosen Produktes erhalten, das nach dem $^1H$-NMR die durchschnittliche Formel

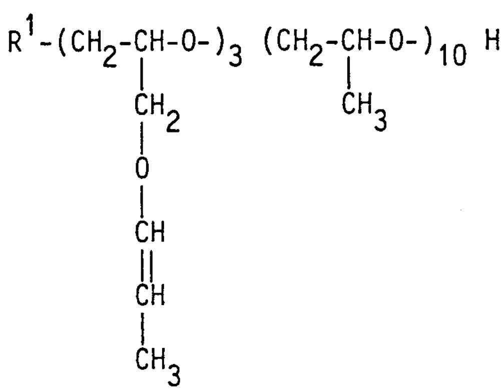

mit

$R^1$ = zu 90 % Butanolrest und
zu 10 % Methanolrest

besitzt.

Beispiel 4

In einem Rührbehälter werden 654 g (1 Mol) eines butanolgestarteten Polyethers mit 10 Propylenoxideinheiten mit 7 g (0,1 Mol) Kaliummethylat vorgelegt, mit einer Stickstoffatmosphäre überlagert und bei 100 °C Methanol abdestilliert. Danach werden 342 g (3 Mol) Propenylglycidether so zugetropft, daß die Temperatur 120 °C nicht überschreitet. Das

Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 926 g (93 % der Theorie) eines leicht gelblichen niedrigviskosen Produktes erhalten, das nach dem $^1$H-NMR die durchschnittliche Formel

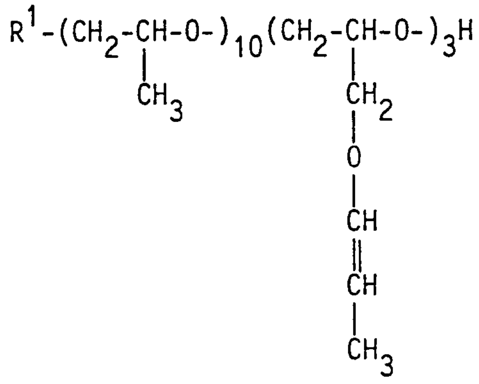

mit

$R^1$ = Butanolrest

besitzt.

Beispiel 5

In einem Druckbehälter werden 66,6 g (0,9 Mol) Butanol mit 7 g (0,1 Mol) Kaliummethylat vorgelegt und mit einer Stickstoffatmosphäre von 2 bar belegt. Bei 100 °C wird gleichzeitig 870 g (15 Mol) Propylenoxid, 660 g (15 Mol) Ethylenoxid und 1140 g (10 Mol) Propenylglycidether so eingeleitet bzw. zugetropft, daß der Druck 5 bar und die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 2575 g (94 % der Theorie) eines leicht gelblichen viskosen Produktes erhalten, das nach dem $^1$H-NMR die durchschnittliche Formel

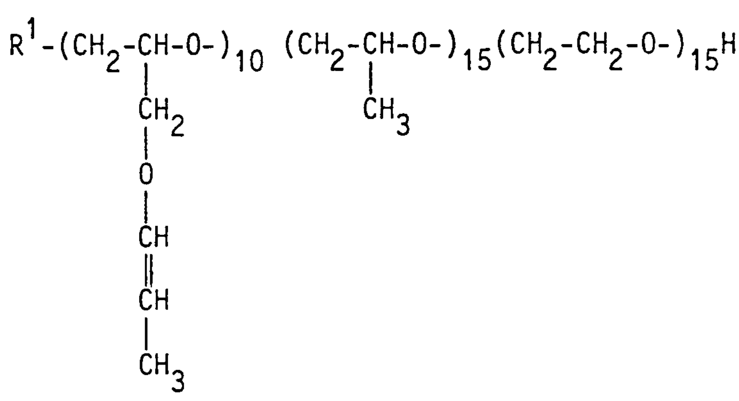

mit

$R^1$ = zu 90 % Butanolrest und
zu 10 % Methanolrest

besitzt.

Beispiel 6

In einem Druckbehälter werden 120,6 g (0,9 Mol) Trimethylolpropan mit 7 g (0,1 Mol) Kaliummethylat vorgelegt, mit einer Stickstoffatmosphäre überlagert und bei 100 °C Methanol abdestilliert. Danach wird mit Stickstoff ein Druck von 2 bar eingestellt und bei 100 °C gleichzeitig 1566 g (27 Mol) Propylenoxid und 615,6 g (5,4 Mol) Propenylglycidether so eingeleitet bzw. zugetropft, daß der Druck 5 bar und die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 2260 g (98 % der Theorie) eines gelblichen mittelviskosen Produktes erhalten, das nach dem $^1$H-NMR die durchschnittliche Formel

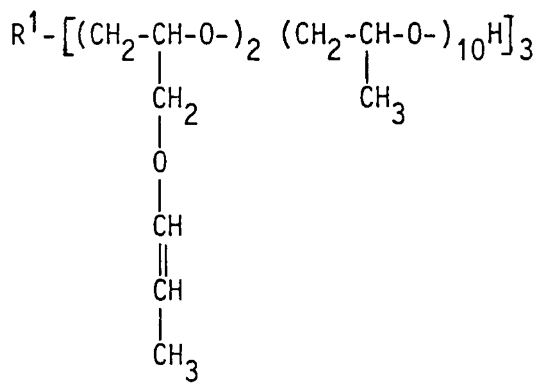

mit

$R^1$ = Trimethylolpropanrest

besitzt.

Beispiel 7

In einem Rührbehälter werden 66,6 g (0,9 Mol) Butanol mit 7 g (0,1 Mol) Kaliummethylat vorgelegt und mit einer Stickstoffatmosphäre überlagert. Bei 100 °C werden 384 g (3 Mol) 1-Methyl-Propenylglycidether so zugetropft, daß die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 408 g (90 % der Theorie) eines leicht gelblichen niedrigviskosen Produktes erhalten, das nach dem $^1$H-NMR die durchschnittliche Formel

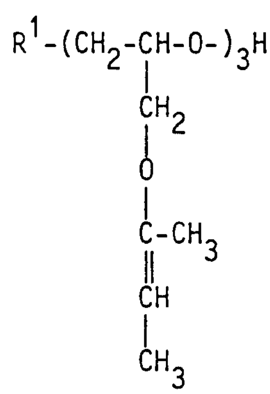

mit

$R^1$ = zu 90 % Butanolrest und
zu 10 % Methanolrest

besitzt.

Beispiel 8

In einem Druckbehälter werden 66,6 g (0,9 Mol) Butanol mit 7 g (0,1 Mol) Kaliummethylat vorgelegt und mit einer Stickstoffatmosphäre von 2 bar belegt. Bei 100 °C werden gleichzeitig 580 g (10 Mol) Propylenoxid und 300 g (3 Mol) Vinylglycidether so eingeleitet bzw. zugetropft, daß der Druck 5 bar und die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Es werden 893 g (94 % der Theorie) eines leicht gelblichen niedrigviskosen Produktes erhalten, das nach dem $^1$H-NMR die durchschnittliche Formel

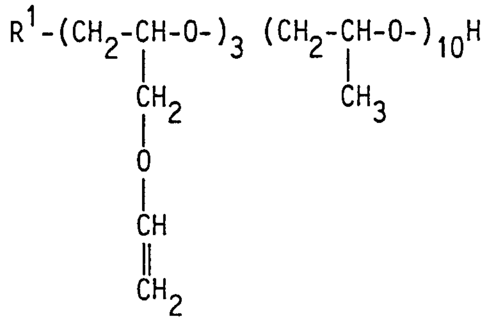

mit

$R^1$ = zu 90 % Butanolrest und
zu 10 % Methanolrest

besitzt.

Beispiel 9

In einem Rührbehälter werden 66,6 g (0,9 Mol) Butanol mit 7 g (0,1 Mol) Kaliummethylat vorgelegt und mit einer Stickstoffatmosphäre überlagert. Bei 100 °C werden 342 g (3 Mol) Allylglycidether so zugetropft, daß die Temperatur 120 °C nicht überschreitet. Das Reaktionsgemisch wird noch 3 Stunden bei 100 °C gerührt und dann mit 13 g 50 %iger Phosphorsäure neutralisiert. Nach Filtration wird das Produkt unter vermindertem Druck bis 100 °C destilliert. Danach wird mit 0,5 g Tris(triphenylphosphin)ruthenium(II)dichlorid über 20 Stunden bei 120 °C gerührt. Es werden 395 g (96 % der Theorie) eines leicht gelblichen niedrigviskosen Produktes erhalten, das nach dem $^1$H-NMR die durchschnittliche Formel

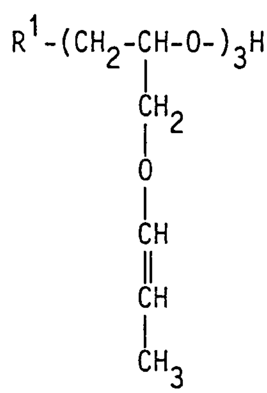

mit

$R^1$ = zu 90 % Butanolrest und zu 10 % Methanolrest

besitzt.

Beispiel 10

In einem Rührbehälter werden 60 g (1 Mol) Ethylendiamin vorgelegt. Danach wird mit Stickstoff gespült. Bei 130 °C werden 501,6 g (4,4 Mol) Propenylglycidether so zugetropft, daß die Temperatur 150 °C nicht überschreitet. Das Reaktionsgemisch wird noch 3 Stunden bei 130 °C gerührt und dann unter vermindertem Druck bis 130 °C destilliert. Es werden 490 g (95 % der Theorie) eines leicht gelblichen niedrigviskosen Produktes erhalten, das nach dem $^1$H-NMR die durchschnittliche Formel

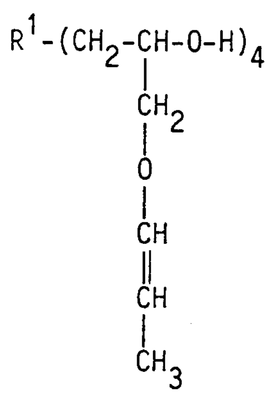

mit

$R^1$ = Ethylendiaminrest

besitzt.

Anwendungstechnische Prüfungen

Die Verbindungen der Beispiele 1 bis 10 werden mit 2 Gew.-% Diphenyliodoniumhexafluorophosphat gut gemischt und mittels einer UV-Lampe der Firma Fusion Systems Corporation, Rockville, MD, USA, Modell I 300 B, im Abstand von 53 mm auf einer Glasplatte in einer Schichtdicke von 20 und 400 µm gehärtet. Die Aushärtungszeiten, die benötigt werden, um eine schmierfreie Oberfläche zu erhalten, sind zusammen mit den Filmeigenschaften in der folgenden Tabelle wiedergegeben:

| Verbindung | Aushärtungszeit (s) | | Filmeigenschaften 400 µm | Bleistifthärte (ECCA-Norm) |
|---|---|---|---|---|
| | 20 µm | 400 µm | | |
| 1 | 2 | 3 | fest | HB |
| 2 | 1 | 3 | hart, spröde | 2H |
| 3 | 2 | 5 | fest, flexibel | 2B |
| 4 | 1 | 4 | fest, flexibel | HB |
| 5 | 1 | 4 | weich, flexibel | 2B |
| 6 | 2 | 5 | fest, hart | 3H |
| 7 | 1 | 3 | fest, hart | H |
| 8 | 1 | 3 | fest, hart | HB |
| 9 | 1 | 3 | fest, hart | H |
| 10* | 2 | 6 | weich, spröde | 2B |

* mit 10 % Photoinitiator

Die Verbindungen der Beispiele 1, 2, 3, 6 und 9 werden zu je 50 % mit Bisphenol-A-diglycidether (Verbindung A) vermischt und mit 4 % Diphenyliodoniumhexafluorophosphat wie beschrieben in einer Schichtdicke von 400 µm auf einer Glasplatte ausgehärtet. Die folgende Tabelle gibt die Aushärtungszeiten und die Filmeigenschaften wieder:

| Mischung mit Verbindung A | Aushärtungszeit (s) | Filmeigenschaften | Bleistifthärte (ECCA-Norm) |
|---|---|---|---|
| Verbindung 1 | 12 | flexibel | 2B |
| Verbindung 2 | 5 | fest | B |
| Verbindung 3 | 8 | weich, flexibel | HB |
| Verbindung 6 | 5 | flexibel | B |
| Verbindung 9 | 4 | flexibel | 2B |
| Verbindung A allein | 30 | weich, flexibel | 3B |

In einem weiteren Versuch wird das als Photoinitiator verwendete Diphenyliodoniumhexafluorophosphat gegen einen Photoinitiator ausgetauscht, der im Handel unter der Bezeichnung NACURE X49-110 erhältlich ist. Es handelt sich dabei um eine blockierte Dinonylnaphthalindisulfonsäure. Sie wird in einer Menge von 5 Gew.-% verwendet und mit den auszuhärtenden Verbindungen vermischt. Die Gemische werden in einer Schichtdicke von 400 µm auf einer Glasplatte bei 120 °C ausgehärtet. In der folgenden Tabelle sind die Zeiten, die zum schmierfilmfreien Aushärten der Mischungen notwendig sind, angegeben. Es sind ferner die Filmeigenschaften und die Härte des Films angegeben.

| Mischung mit Verbindung A | Aushärtungszeit (min) | Filmeigenschaften | Bleistifthärte (ECCA-Norm) |
|---|---|---|---|
| Verbindung 1 | 22 | fest | HB |
| Verbindung 2 | 15 | hart | H |
| Verbindung 3 | 16 | fest, flexibel | HB |
| Verbindung 6 | 15 | hart | 2H |
| Verbindung 9 | 12 | hart | H |
| Verbindung A allein | 30 | hart | H |

## Patentansprüche

1. Kationisch härtbare Oxyalkylenether der allgemeinen durchschnittlichen Formel

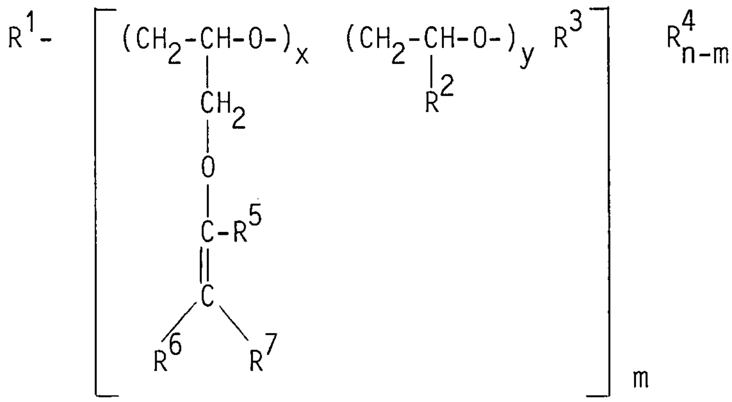

| | |
|---|---|
| $R^1$ | = n-wertiger Rest einer zur Anlagerung von Oxiranen befähigten Verbindung $(R^1)H_n$ mit n aktiven Wasserstoffatomen oder einwertiger Alkyl- oder Arylrest, $n \geqq 1$, |
| $R^2$ | = Wasserstoff-, Kohlenwasserstoff- oder $R^3OCH_2$-Rest, |
| $R^3$, $R^4$ | = Wasserstoff-, Kohlenwasserstoff- oder Acylreste, wobei die Reste $R^1$, $R^2$, $R^3$ und $R^4$ im Molekül unterschiedliche Bedeutung haben können, |
| $R^5$, $R^6$ $R^7$ | = Wasserstoff- oder Alkylreste mit 1 bis 8 Kohlenstoffatomen oder $R^5$ und $R^7$ oder $R^6$ und $R^7$ gemeinsam Bestandteil eines cyclischen, nichtaromatischen Kohlenwasserstoffrestes mit 5 oder 6 Kohlenstoffatomen, |
| m | mindestens = 1 und höchstens = n |
| x | im durchschnittlichen Molekül = 1 bis 100, |
| y | im durchschnittlichen Molekül = 0 bis 100, wobei $1 \leqq x + y < 150$ ist, |

wobei Oxyalkylenether ausgenommen sind, bei denen
$R^1$ = $-O-n-C_{16}H_{33}$,
$R^3$ = H oder $-CH_2-CH=CH-CH_3$,
$R^5 = R^6 = H$,
$R^7 = CH_3$ und
x = 1, y = 0; m = 1 ist.

2. Oxyalkylenether nach Anspruch 1, dadurch gekennzeichnet, daß mindestens einer der Reste $R^5$, $R^6$ und $R^7$ ein Alkylrest ist.

3. Oxyalkylenether nach Anspruch 1 oder 2, dadurch gekennzeichnet, daß der Rest $R^6$ und/oder $R^7$ ein Alkylrest ist.

4. Oxyalkylenether nach einem oder mehreren der vorhergehenden Ansprüche, dadurch gekennzeichnet, daß $R^5$ und $R^6$ Wasserstoffreste sind und $R^7$ ein Methylrest ist.

5. Oxyalkylenether nach einem oder mehreren der vorhergehenden Ansprüche, dadurch gekennzeichnet, daß $R^5$ und $R^7$ gemeinsam Bestandteil eines cyclischen, nichtaromatischen Kohlenwasserstoffrestes mit 5 oder 6 Kohlenstoffatomen sind und $R^6$ ein Wasserstoffrest ist.

6. Oxyalkylenether nach einem oder mehreren der vorhergehenden Ansprüche, dadurch gekennzeichnet, daß $1 < x + y < 150$ ist.

7. Oxyalkylenether nach einem oder mehreren der vorhergehenden Ansprüche, dadurch gekennzeichnet, daß x einen durchschnittlichen Wert von 1 bis 25 hat.

8. Oxyalkylenether nach einem oder mehreren der vorhergehenden Ansprüche, dadurch gekennzeichnet, daß die Verbindung $(R^1)H_n$ Wasser, Ammoniak, ein ein- oder mehrwertiger Alkohol, ein ein- oder mehrwertiges Phenol, eine ein- oder mehrwertige Carbonsäure oder ein ein- oder mehrwertiges Amin ist.

9. Oxyalkylenether nach einem oder mehreren der vorhergehenden Ansprüche, dadurch gekennzeichnet, daß die Verbindung $(R^1)H_n$ ein einwertiger gesättigter aliphatischer Alkohol mit 1 bis 13 Kohlenstoffatomen ist.

10. Oxyalkylenether nach einem oder mehreren der vorhergehenden Ansprüche, dadurch gekennzeichnet, daß die Verbindung $(R^1)H_n$ ein einwertiger ungesättigter Alkohol mit 3 bis 13 Kohlenstoffatomen ist.

11. Oxyalkylenether nach einem oder mehreren der vorhergehenden Ansprüche, dadurch gekennzeichnet, daß die Verbindung $(R^1)H_n$ ein zwei- bis sechswertiger aliphatischer Alkohol mit 2 bis 6 Kohlenstoffatomen ist.

12. Oxyalkylenether nach einem oder mehreren der vorhergehenden Ansprüche, dadurch gekennzeichnet, daß die Verbindung $(R^1)H_n$ eine aliphatische, gesättigte oder ungesättigte ein- oder mehrwertige Carbonsäure ist.

13. Oxyalkylenether nach einem oder mehreren der vorhergehenden Ansprüche, dadurch gekennzeichnet, daß die Verbindung $(R^1)H_n$ ein aliphatisches Mono- oder Diamin mit 1 bis 7 Kohlenstoffatomen ist.

14. Oxyalkylenether nach einem oder mehreren der vorhergehenden Ansprüche, dadurch gekennzeichnet, daß der durchschnittliche Wert des Produktes m . x 2 bis 25 und der durchschnittliche Wert des Produktes m . y 0 bis 25 ist.

15. Oxyalkylenether nach einem oder mehreren der vorhergehenden Ansprüche, dadurch gekennzeichnet, daß $R^2$ ein Wasserstoff-, Methyl-, Ethyl- oder $R^3OCH_2$-Rest ist, wobei $R^3$ ein Wasserstoff- oder Alkylrest ist.

16. Verfahren zur Herstellung der Verbindungen nach einem oder mehreren der Ansprüche 1 bis 15, dadurch gekennzeichnet, daß man x . m Mol Monomere der allgemeinen Formel

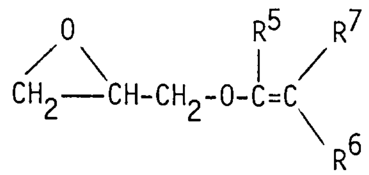

und gegebenenfalls y . m Mol Monomere der allgemeinen Formel

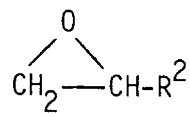

an 1 Mol einer Verbindung der allgemeinen Formel $(R^1)H_n$ statistisch oder blockweise unter alkalischen oder neutralen Bedingungen anlagert und gegebenenfalls die erhaltenen Verbindungen ganz oder teilweise mit Alkylhalogeniden der Formel $R^3X$ verethert oder mit Carbonsäureanhydriden der Formel $(R^3CO)_2O$ oder Acylhalogeniden der Formel $R^3COX$ verestert, wobei X ein Halogenatom ist.

**17.** Verfahren zur Herstellung der Verbindungen nach einem oder mehreren der Ansprüche 2 bis 15, dadurch gekennzeichnet, daß man Verbindungen der allgemeinen Formel

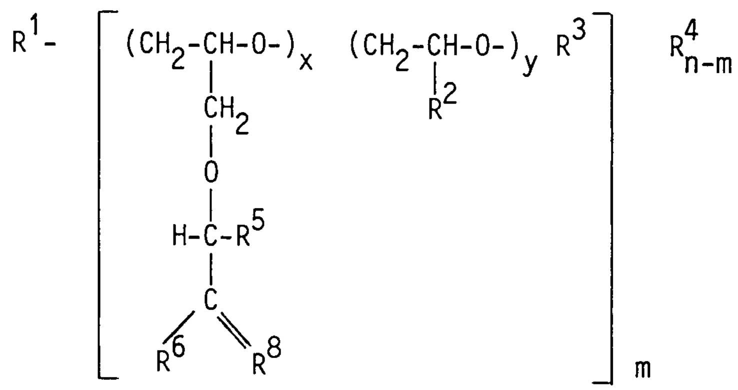

wobei $R^1$, $R^2$, $R^3$, $R^4$, $R^5$ und $R^6$ die bereits angegebenen Bedeutungen und x, y, n und m die bereits angegebenen Werte haben und der Rest $R^8$ ein Kohlenwasserstoffrest mit 1 bis 8 Kohlenstoffatomen und zwei freien Bindungen an einem Kohlenstoffatom ist, in an sich bekannter Weise umlagert.

**18.** Verfahren nach Anspruch 17, dadurch gekennzeichnet, daß man die Umlagerung durch Einwirkung von Alkalialkoholen oder Metallkomplexen oder Übergangsmetalle enthaltenden Katalysatoren bei Temperaturen von 25 bis 170 °C, gegebenenfalls in polaren Lösungsmitteln, vornimmt.

**19.** Verwendung der Verbindungen nach einem der Patentansprüche 1 bis 18 als gegebenenfalls photochemisch härtbare Oligomere in Vergußmassen oder Beschichtungsmitteln oder als reaktive Verdünnungsmittel für Epoxidharze.

## Claims

**1.** Cationically curable oxyalkylene ethers of the general average formula

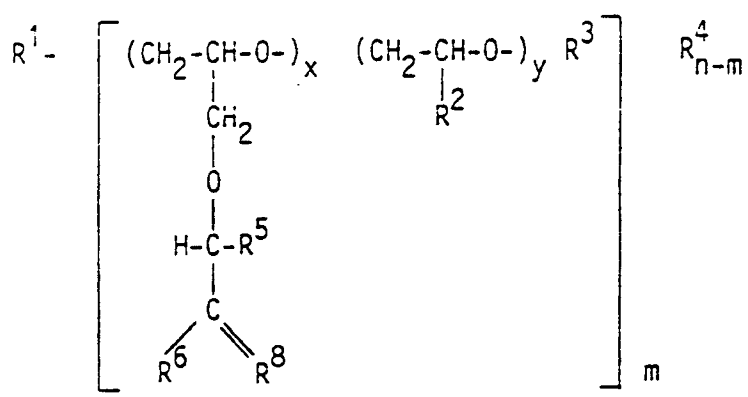

| | |
|---|---|
| $R^1$ | is an n-valent radical of a compound $(R^1)H_n$ containing n active hydrogen atoms which is capable of adduction by oxiranes, or is a monovalent alkyl or aryl radical, n is $\geqq 1$, |
| $R^2$ | is a hydrogen, hydrocarbon or $R^3OCH_2$ radical, |
| $R^3$ and $R^4$ | are hydrogen, hydrocarbon or acyl radicals, where the radicals $R^1$, $R^2$, $R^3$ and $R^4$ can have different meanings in the molecule, |
| $R^5$, $R^6$ and $R^7$ | are hydrogen or alkyl radicals having 1 to 8 carbon atoms or $R^5$ and $R^7$ or $R^6$ and $R^7$ together are part of a cyclic, nonaromatic hydrocarbon radical having 5 or 6 carbon atoms, |
| m | is at least 1 and at most n, |
| x | in the average molecule is from 1 to 100, |
| y | in the average molecule is from 0 to 100, where $1 \leqq x + y < 150$, |

with the exception of oxyalkylene ethers in which
$R^1$ is $-O-n-C_{16}H_{33}$,
$R^3$ is H or $-CH_2-CH=CH-CH_3$,
$R^5$ and $R^6$ are H,
$R^7$ is $CH_3$ and
$x = 1$, $y = 0$ and $m = 1$.

**2.** Oxyalkylene ethers according to Claim 1, characterized in that at least one of the radicals $R^5$, $R^6$ and $R^7$ is an alkyl radical.

**3.** Oxyalkylene ethers according to Claim 1 or 2, characterized in that the radical $R^6$ and/or $R^7$ is an alkyl radical.

**4.** Oxyalkylene ethers according to one or more of the preceding claims, characterized in that $R^5$ and $R^6$ are hydrogen radicals and $R^7$ is a methyl radical.

**5.** Oxyalkylene ethers according to one or more of the preceding claims, characterized in that $R^5$ and $R^7$ together are part of a cyclic, nonaromatic hydrocarbon radical having 5 or 6 carbon atoms, and $R^6$ is a hydrogen radical.

**6.** Oxyalkylene ethers according to one or more of the preceding claims, characterized in that $1 < x + y < 150$.

**7.** Oxyalkylene ethers according to one or more of the preceding claims, characterized in that x has an average value of from 1 to 25.

**8.** Oxyalkylene ethers according to one or more of the preceding claims, characterized in that the compound $(R^1)H_n$ is water, ammonia, a monohydric or polyhydric alcohol, a monohydric or polyhydric phenol, a monobasic or polybasic carboxylic acid or a monoamine or polyamine.

**9.** Oxyalkylene ethers according to one or more of the preceding claims, characterized in that the compound $(R^1)H_n$ is a monohydric saturated aliphatic alcohol having 1 to 13 carbon atoms.

**10.** Oxyalkylene ethers according to one or more of the preceding claims, characterized in that the compound $(R^1)H_n$ is a monohydric unsaturated alcohol having 3 to 13 carbon atoms.

**11.** Oxyalkylene ethers according to one or more of the preceding claims, characterized in that the compound $(R^1)H_n$ is a dihydric to hexahydric aliphatic alcohol having 2 to 6 carbon atoms.

**12.** Oxyalkylene ethers according to one or more of the preceding claims, characterized in that the compound $(R^1)H_n$ is an aliphatic, saturated or unsaturated, monobasic or polybasic carboxylic acid.

**13.** Oxyalkylene ethers according to one or more of the preceding claims, characterized in that the compound $(R^1)H_n$ is an aliphatic monoamine or diamine having 1 to 7 carbon atoms.

**14.** Oxyalkylene ethers according to one or more of the preceding claims, characterized in that the average value of the product m . x is from 2 to 25, and the average value of the product m . y is from 0 to 25.

**15.** Oxyalkylene ethers according to one or more of the preceding claims, characterized in that $R^2$ is a hydrogen, methyl, ethyl or $R^3OCH_2$ radical, where $R^3$ is a hydrogen or alkyl radical.

**16.** Process for the preparation of the compounds according to one or more of Claims 1 to 15, characterized in that x . m mol of monomers of the general formula

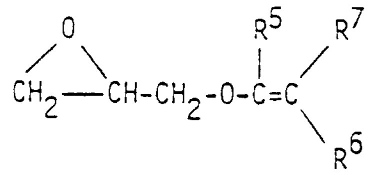

and, if desired, y . m mol of monomers of the general formula

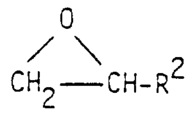

are added onto 1 mol of a compound of the general formula $(R^1)H_n$ in a random or blockwise manner under alkaline or neutral conditions, and, if desired, the resultant compounds are fully or partially etherified using alkyl halides of the formula $R^3X$ or esterified using carboxylic anhydrides of the formula $(R^3CO)_2O$ or acyl halides of the formula $R^3COX$, where X is a halogen atom.

**17.** Process for the preparation of the compounds according to one or more of Claims 2 to 15, characterized in that compounds of the general formula

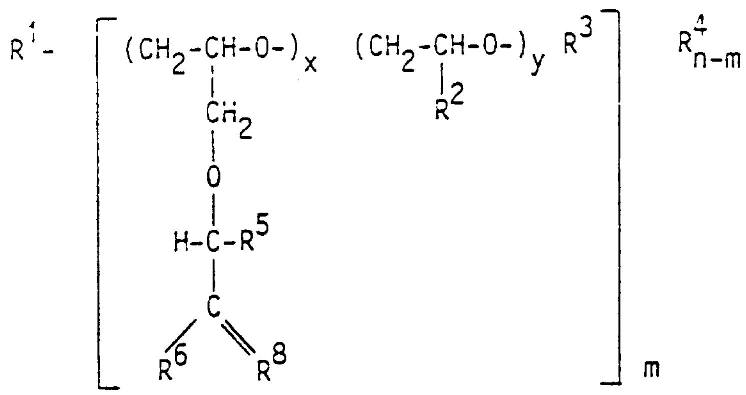

where $R^1$, $R^2$, $R^3$, $R^4$, $R^5$ and $R^6$ are as defined above, and x, y, n and m have the values defined above, and the radical $R^8$ is a hydrocarbon radical having 1 to 8 carbon atoms and containing two free bonds to a carbon atom, are rearranged in a manner known per se.

**18.** Process according to Claim 17, characterized in that rearrangement is carried out by the action of catalysts containing transition metals or alkali metal alkoxides or metal complexes at a temperature of from 25 to 170°C, optionally in polar solvents.

**19.** Use of the compounds according to one of Claims 1 to 18 as optionally photochemically curable oligomers in encapsulation compositions or coating compositions or as reactive diluents for epoxy resins.

## Revendications

1. Ethers d'oxyalkylène à durcissement cationique, répondant à la formule générale moyenne :

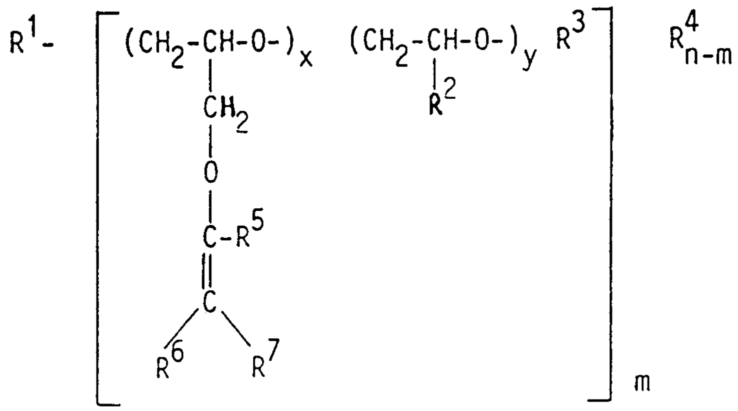

| | |
|---|---|
| $R^1$ | = un reste de valence n d'un composé $(R^1)H_n$ capable de fixer des oxirannes par addition, avec n atomes d'hydrogène actif, ou un reste alkyle ou aryle monovalent, $n \geqq 1$, |
| $R^2$ | = un reste d'hydrogène, un reste hydrocarboné ou le reste $R^3OCH_2$, |
| $R^3$, $R^4$ | = des restes d'hydrogène, des restes hydrocarbonés ou des restes acyles, les restes $R^1$, $R^2$, $R^3$ et $R^4$ pouvant avoir des significations différentes dans la molécule, |
| $R^5$, $R^6$, $R^7$ | = des restes d'hydrogène ou des restes alkyles ayant de 1 à 8 atomes de carbone, ou $R^5$ et $R^7$ ou $R^6$ et $R^7$ pris ensemble forment un constituant d un reste hydrocarboné cyclique non aromatique ayant 5 ou 6 atomes de carbone, |
| m | = au moins 1 et au plus n, |
| x | = 1 à 100 dans la molécule moyenne, |
| y | = 0 à 100 dans la molécule moyenne, avec $1 \leqq x + y < 150$, |

à l'exception des éthers d'oxyalkylène dans lesquels
$R^1 = -O-n-C_{16}H_{33}$,
$R^3 = H$ ou $-CH_2-CH=CH-CH_3$,
$R^5 = R^6 = H$,
$R^7 = CH_3$, et
$x = 1$ ; $y = 0$ ; $m = 1$.

2. Ethers d'oxyalkylène selon la revendication 1, caractérisés en ce qu'au moins un des restes $R^5$, $R^6$ et $R^7$ est un reste alkyle.

3. Ethers d'oxyalkylène selon la revendication 1 ou 2, caractérisés en ce que le reste $R^6$ et/ou le reste $R^7$ est un reste alkyle.

4. Ethers d'oxyalkylène selon une ou plusieurs des revendications précédentes, caractérisés en ce que $R^5$ et $R^6$ sont des restes d'hydrogène, et $R^7$ est un reste méthyle.

5. Ethers d'oxyalkylène selon une ou plusieurs des revendications précédentes, caractérisés en ce que $R^5$ et $R^7$ pris ensemble forment un constituant d'un reste hydrocarboné cyclique non aromatique ayant 5 ou 6 atomes de carbone, et $R^6$ est un reste d'hydrogène.

6. Ethers d'oxyalkylène selon une ou plusieurs des revendications précédentes, caractérisés en ce que $1 < x + y < 150$.

7. Ethers d'oxyalkylène selon une ou plusieurs des revendications précédentes, caractérisés en ce que x a une valeur moyenne de 1 à 25.

**8.** Ethers d'oxyalkylène selon une ou plusieurs des revendications précédentes, caractérisés en ce que le composé $(R^1)H_n$ est l eau, l'ammoniaque, un alcool mono- ou polyvalent, un phénol mono- ou polyvalent, un acide carboxylique mono- ou polyvalent, ou une amine mono- ou polyvalente.

**9.** Ethers d'oxyalkylène selon une ou plusieurs des revendications précédentes, caractérisés en ce que le composé $(R^1)H_n$ est un alcool aliphatique monovalent saturé ayant de 1 à 13 atomes de carbone.

**10.** Ethers d'oxyalkylène selon une ou plusieurs des revendications précédentes, caractérisés en ce que le composé $(R^1)H_n$ est un alcool monovalent insaturé ayant de 3 à 13 atomes de carbone.

**11.** Ethers d'oxyalkylène selon une ou plusieurs des revendications précédentes, caractérisés en ce que le composé $(R^1)H_n$ est un alcool aliphatique bivalent à hexavalent ayant de 2 à 6 atomes de carbone.

**12.** Ethers d'oxyalkylène selon une ou plusieurs des revendications précédentes, caractérisés en ce que le composé $(R^1)H_n$ est un acide carboxylique aliphatique mono- ou polyvalent, saturé ou insaturé.

**13.** Ethers d'oxyalkylène selon une ou plusieurs des revendications précédentes, caractérisés en ce que le composé $(R^1)H_n$ est une monoamine ou une diamine aliphatique ayant de 1 à 7 atomes de carbone.

**14.** Ethers d'oxyalkylène selon une ou plusieurs des revendications précédentes, caractérisés en ce que la valeur moyenne du produit m . x est de 2 à 25, et la valeur moyenne du produit m . y est de 0 à 25.

**15.** Ethers d'oxyalkylène selon une ou plusieurs des revendications précédentes, caractérisés en ce que $R^2$ est un reste d'hydrogène, un reste méthyle, éthyle ou $R^3OCH_2$, où $R^3$ est un reste d'hydrogène ou un reste alkyle.

**16.** Procédé de préparation des composés selon une ou plusieurs des revendications 1 à 15, caractérisé en ce qu'on fixe par addition x.m moles de monomères répondant à la formule générale

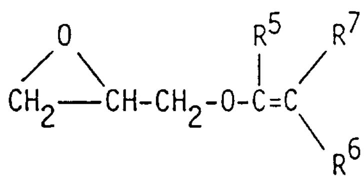

et éventuellement y.m moles de monomères répondant à la formule générale

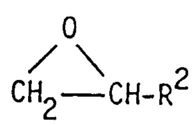

sur 1 mole d'un composé de formule générale $(R^1)H_n$, de manière statistique ou en bloc, dans des conditions alcalines ou neutres, et éventuellement, on éthérifie les composés obtenus, complètement ou partiellement, avec des halogénures d'alkyle répondant à la formule $R^3X$ ou on les estérifie avec des anhydrides d'acide carboxylique répondant à la formule $(R^3CO)_2O$ ou avec des halogénures d'acyle répondant a la formule $R^3COX$, X étant un atome d'halogène.

**17.** Procédé de préparation des composés selon une ou plusieurs des revendications 2 à 15, caractérisé en ce qu'on réarrange des composés répondant à la formule générale

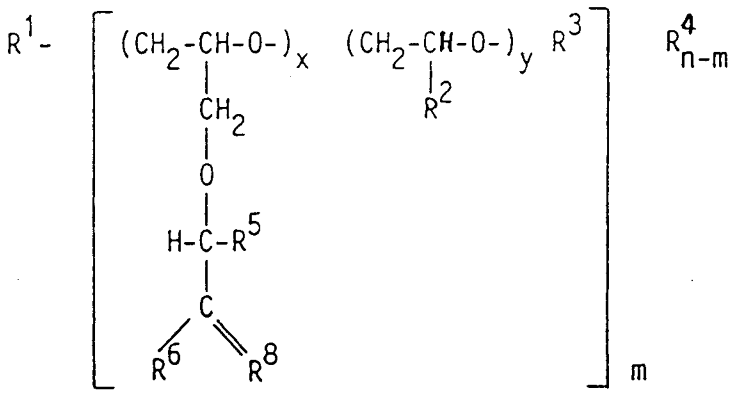

où $R^1$, $R^2$, $R^3$, $R^4$, $R^5$ et $R^6$ ont les significations déjà indiquées, et x, y, n et m ont les valeurs déjà indiquées, et le reste $R^8$ est un reste hydrocarboné ayant de 1 à 8 atomes de carbone et deux liaisons libres sur un atome de carbone, d'une manière connue en soi.

**18.** Procédé selon la revendication 17, caractérisé en ce qu'on effectue le réarrangement sous l'action d'alcools alcalins ou de complexes de métaux ou de catalyseurs contenant des métaux de transition, à des températures de 25 à 170°C, éventuellement dans des solvants polaires.

**19.** Utilisation des composés selon l'une des revendications 1 à 18 comme des oligomères éventuellement durcissables de manière photochimique, dans des masses de remplissage ou des agents de revêtements ou comme des diluants réactifs pour des résines époxydes.